# FASCICULE DE BREVET EUROPEEN

(11) **EP 0 776 977 B1**
(45) Date de publication et mention de la délivrance du brevet: **27.05.1998**
(21) Numéro de dépôt: 96402298.2
(22) Date de dépôt: 29.10.1996
(51) Int. Cl.: C12P 17/10, C07D 209/08

(54) **Procédé de préparation de composés indoliques**
Herstellungsverfahren von Indol-Verbindungen
Process for the preparation of indol compounds

(30) Priorité: 06.12.1995 FR 9514373
(43) Date de publication de la demande: 04.06.1997
(73) Titulaire: L'OREAL, 75008 Paris (FR)
(72) Inventeur: Lagrange, Alain, 77450 Coupvray (FR); Philippe, Michel, 91320 Wissous (FR); Tuloup, Rémy, 35190 Miniac sous Becherel (FR)
(74) Mandataire: Miszputen, Laurent

(56) Documents cités:
- EP-A- 0 682 014
- WO-A-95/03421
- FR-A- 2 649 009
- GB-A- 981 192
- Enzyme Nomenclature, Recommendations (1972) of the Commission of Biochemical Nomenclature on the Nomenclature and Classification of Enzymes, 1975, Elsevier, NL, pages 20-21

## Description

La présente invention concerne un nouveau procédé de préparation de composés indoliques par voie enzymatique.

L'indole et ses dérivés sont des matières premières largement utilisées dans l'industrie des colorants, des cosmétiques, des produits pharmaceutiques ou alimentaires. L'indole est, en particulier, une des matières premières utilisables pour la synthèse du tryptophane qui est un acide aminé essentiel sur le plan nutritionnel. Les composés indoliques peuvent être, en particulier, utilisés pour la teinture des fibres kératiniques, comme cela est par exemple décrit dans la demande de brevet FR-A-2 649 009.

La synthèse des indoles se fait en général en plusieurs étapes, de façon longue et coûteuse. De plus, cette synthèse nécessite des étapes d'oxydation poussées, à des températures de réaction élevées, souvent supérieures à 140 °C, entraînant des risques de manipulation.

C'est ainsi qu'ont été proposés différents procédés de préparation de composés indoliques tel que décrit par exemple dans la demande de brevet FR-A-2 606 405, opérant soit par hydrogénation catalytique sous pression, soit par transfert d'hydrogène ou encore par cyclisation réductrice catalytique tel que décrit par exemple dans le brevet US 4 595 765.

Ces méthodes nécessitent l'utilisation de précurseurs d'indole portant des groupements compatibles avec une réaction d'hydrogénation, ce qui entraîne une complication du procédé en amont. De plus ces procédés conduisent généralement à l'obtention de produits secondaires affectant la pureté de la réaction.

Il a également été proposé de préparer des composés indoliques par déshydrogénation de dérivés d'indolines tel que décrit par exemple dans la demande de brevet DE-A-4 204 089 ou bien encore par hydrolyse basique à partir de 5,6-diacétate d'indole tel que décrit par exemple par R. J. S. BEER et autres dans "Journal of Chemical Society", 2223-2226, 1948.

L'inconvénient de ces deux dernières méthodes est que la réaction se déroule préférentiellement dans l'eau en présence d'un agent alcalin, ce qui entraîne généralement une dégradation rapide du composé indolique obtenu.

La synthèse du 5,6-dihydroxy indole à partir de 3,4-dihydroxyphénylalanine (DOPA) par voie chimique est également connue et décrite par exemple dans la demande de brevet EP-A-0 682 014.

C'est en cherchant à remédier à ces problèmes que la demanderesse a découvert le procédé objet de l'invention.

La présente invention a donc pour objet un nouveau procédé de préparation de composés indoliques en une seule étape, qui est caractérisé par le fait qu'il consiste à mettre en contact, en milieu solvant, au moins une hydrolase avec au moins un composé indolique portant un ou deux radicaux reliés par une fonction ester au cycle benzénique, ce par quoi l'on obtient un mono- ou un dihydroxyindole.

Selon ce procédé, il ne se forme pas de produit secondaire, ce qui présente un intérêt tant du point de vue de la pureté du produit final obtenu que du rendement de la réaction en ce produit. De plus, ce procédé de synthèse simple ne nécessite pas l'emploi d'agent alcalin dans le milieu réactionnel, ce qui permet d'éviter la dégradation rapide du composé indolique obtenu.

Selon le procédé conforme à l'invention, les composés indoliques de départ portant un ou deux radicaux reliés par une fonction ester au cycle benzénique sont de préférence choisis parmi les composés de formule (I) suivante : 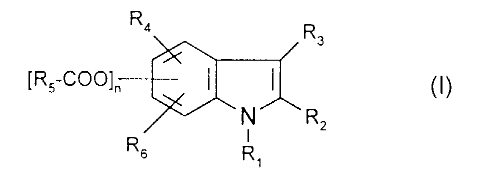 dans laquelle :
- n est un nombre entier égal à 1 ou 2 ;
- R₁ représente un atome d'hydrogène ou un radical alkyle en C₁-C₄ ;
- R₂, R₃, R₄ et R₆, identiques ou différents, représentent un atome d'hydrogène, un radical alkyle en C₁-C₈, alcoxy(C₁-C₄)alkyle en C₁-C₄, alcoxy en C₁-C₈, alcoxy(C₁-C₄)alcoxy en C₁-C₄, aryle en C₅-C₁₀, aryloxy en C₅-C₁₀, aryle en C₅-C₁₀ comportant de 1 à 3 hétéroatomes tel qu'un atome d'oxygène ou d'azote, aryloxy en C₅-C₁₀ comportant de 1 à 3 hétéroatomes tel qu'un atome d'oxygène ou d'azote, acyle en C₁-C₈, ou bien un radical pris dans le groupe constitué par les radicaux suivants : 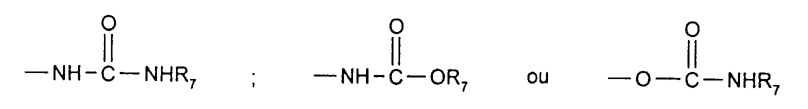 dans lesquels le radical R₇ représente un radical alkyle en C₁-C₈ ;
- R₅ représente un radical alkyle en C₁-C₈, un radical aryle en C₅-C₁₀, aryl(C₅-C₁₀)alkyle en C₁-C₈, alkyl(C₁-C₈)aryle en C₅-C₁₀ ou acyl(C₂-C₈)aryle en C₅-C₁₀.

Dans la formule (I) ci-dessus, les radicaux alkyle et alcoxy peuvent être linéaires ou ramifiés, saturés ou insaturés.

Le radical acyle désigne de préférence un radical alcanoyle dérivé d'un acide carboxylique aliphatique ayant de 5 à 10 atomes de carbone tels que par exemple les groupes formyle, acétyle ; ou un groupe aroyle dérivé d'un acide carboxylique aromatique tel que le groupe benzoyle.

Les mono- et dihydroxyindoles pouvant être obtenus par le procédé conforme à l'invention lorsqu'appliqué aux composés de départ de formule (I) ci-dessus répondent donc à la formule (II) suivante : 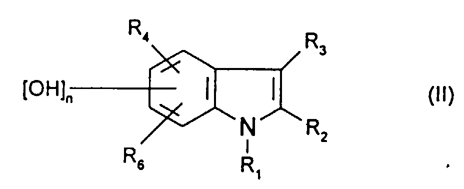 dans laquelle :
- n est un nombre entier égal à 1 ou 2 ;
- et R₁, R₂, R₃, R₄ et R₆ ont les mêmes significations que dans la formule (I).

Parmi les composés indoliques que l'on peut obtenir en mettant en oeuvre le procédé de l'invention, on peut citer :
- le 5,6-dihydroxyindole,
- le 6-hydroxyindole,
- le 4-hydroxy indole,
- le 5-hydroxyindole,
- le 7-hydroxyindole,
- le 5,7-dihydroxyindole.

Des composés particulièrement préférés sont :
- le 5,6-dihydroxyindole,
- le 6-hydroxyindole,
- le 4-hydroxy indole,
- le 7-hydroxyindole.

Selon le procédé de l'invention, la nature de l'hydrolase n'est pas critique. Par hydrolase, on entend toute substance enzymatique qui sous l'action de l'eau entraîne la coupure d'une liaison ester. Ces hydrolases sont par exemple décrites par H.G. Davis, R.H. Green, D.R. Kelly et S.M. Roberts dans "Biotransformations in preparative organic chemistry", Academic Press, 1989.

Parmi les hydrolases utilisables selon l'invention, on peut en particulier citer les lipases, les estérases et les protéases. Ces hydrolases peuvent éventuellement être fixées (supportées) sur des supports inertes tels que des billes de verre, de la célite, de l'argile ou de la résine.

A titre de lipase, on peut tout particulièrement citer la lipase de Pseudomonas Fluorescens ainsi que la lipase de Candida Antartica fixée sur résine et commercialisée sous la dénomination Novozym 435 par la société Novo-Nordisk.

Selon le procédé de l'invention, la ou les hydrolases représentent de préférence de 5 % à 500 % en poids environ par rapport au poids du composé indolique de départ à faire réagir et encore plus préférentiellement de 10 % à 100 % en poids environ de ce poids.

Selon un mode de réalisation préféré du procédé selon l'invention, le pH du milieu solvant est compris entre 5 et 9 environ et encore plus préférentiellement est compris entre 6,5 et 7,5 environ.

Afin d'éviter les variations de pH en cours de réaction, on ajoute de préférence au milieu réactionnel, une solution tampon classique telle que par exemple le tampon phosphate.

D'une manière avantageuse, le solvant utilisé est un solvant inerte comme par exemple un alcool inférieur en C₁-C₄ tel que le méthanol, l'éthanol ou l'isopropanol ; l'acétonitrile ; le tetrahydrofurane ; le toluène ; l'hexane ; l'heptane, le chloroforme ou le dichlorométhane, en présence ou non d'un agent de transfert de phase tel que par exemple un ammonium quaternaire ou un sel d'acide sulfosuccinique. De façon préférentielle, le solvant utilisé est un alcool en C₁-C₄ tel que l'éthanol.

Selon l'invention, la température de la réaction n'est pas critique mais il faut que cette température soit compatible avec un bon fonctionnement de l'enzyme utilisée.

Selon un forme de réalisation avantageuse du procédé de l'invention, la température de la réaction est comprise entre 20 et 80°C environ et de préférence entre 25 et 50°C environ. Une température tout particulièrement préférée se situe aux environs de 37°C.

Lorsque la réaction est terminée, les produits attendus, tels que par exemple le 5,6-dihydroxyindole, peuvent, le cas échéant, être récupérés par des méthodes bien connues de l'état de la technique telles que la filtration ou cristallisation.

Les composés indoliques obtenus selon le procédé conforme à l'invention peuvent également être ensuite purifiés selon les méthodes classiques et bien connues de l'homme du métier, telles que par exemple la chromatographie sur gel de silice.

Les produits indoliques obtenus selon le procédé conforme à l'invention peuvent être utilisés à de nombreuses fins, par exemple comme composés intermédiaires dans la préparation d'acides aminés, d'alcaloïdes de tryptamines, ou encore comme produits finis dans tout type d'industrie, chimique, cosmétique, alimentaire, pharmaceutique, ou autre.

Les exemples de préparation qui suivent sont destinés à illustrer l'invention sans pour autant en limiter la portée.

### EXEMPLES

### EXEMPLE 1 : Synthèse du 5,6-dihydroxyindole

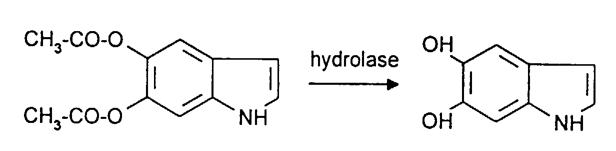

Dans un Erlenmeyer de 500 ml, on a mélangé 6 g de 5,6-diacétoxy indole, 4 g de Novozym 435 commercialisé par la société Novo-Nordisk (hydrolase), 100 ml d'éthanol à 95° et 100 ml de tampon phosphate 0,1M à pH 7.

Au bout de 2 heures et 30 minutes d'agitation à une température d'environ 37°C, le milieu réactionnel a été filtré, évaporé à sec puis repris par 50 ml d'acétate d'éthyle.

La solution obtenue a ensuite été chromatographiée sur gel de silice, sous pression d'azote en prenant comme éluant un mélange acétate d'éthyle heptane (20/80).

Après élimination du 5,6-diacétoxy indole n'ayant pas réagi, on a récupéré le produit attendu dont l'analyse élémentaire pour C₈H₇NO₂ était la suivante :

| **%** | **C** | **H** | **N** | **O** |
|---|---|---|---|---|
| **Calculée** | 64,43 | 4,70 | 9,39 | 21,47 |
| **Trouvée** | 64,18 | 4,89 | 9,22 | 21,46 |

## Revendications

1. Procédé de préparation de composés indoliques en une seule étape, caractérisé par le fait qu'il consiste à mettre en contact, en milieu solvant, au moins une hydrolase choisie parmi les substances enzymatiques qui sous l'action de l'eau entraînent la coupure d'une liaison ester, avec au moins un composé indolique portant un ou deux radicaux reliés par une fonction ester au cycle benzénique, ce par quoi l'on obtient un mono- ou un dihydroxyindole.

2. Procédé selon la revendication 1, caractérisé par le fait que les composés indoliques de départ portant un ou deux radicaux reliés par une fonction ester au cycle benzénique sont choisis parmi les composés de formule (I) suivante : 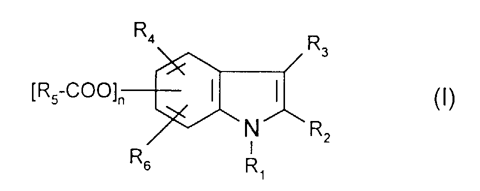 dans laquelle :
- n est un nombre entier égal à 1 ou 2 ;
- R₁ représente un atome d'hydrogène ou un radical alkyle en C₁-C₄
- R₂, R₃, R₄ et R₆, identiques ou différents, représentent un atome d'hydrogène, un radical alkyle en C₁-C₈, alcoxy(C₁-C₄)alkyle en C₁-C₄, alcoxy en C₁-C₈, alcoxy(C₁-C₄)alcoxy en C₁-C₄, aryle en C₅-C₁₀, aryloxy en C₅-C₁₀, aryle en C₅-C₁₀ comportant de 1 à 3 hétéroatomes tel qu'un atome d'oxygène ou d'azote, aryloxy en C₅-C₁₀ comportant de 1 à 3 hétéroatomes tel qu'un atome d'oxygène ou d'azote, acyle en C₁-C₈, ou bien un radical pris dans le groupe constitué par les radicaux suivants : 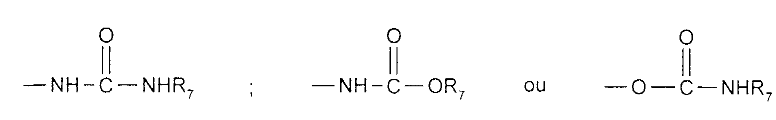 dans lesquels le radical R₇ représente un radical alkyle en C₁-C₈ ;
- R₅ représente un radical alkyle en C₁-C₈, un radical aryle en C₅-C₁₀, aryl(C₅-C₁₀)alkyle en C₁-C₈, alkyl(C₁-C₈)aryle en C₅-C₁₀ ou acyl(C₂-C₈)aryle en C₅-C₁₀.

3. Procédé selon la revendication 2, caractérisé par le fait que dans la formule (I), les radicaux alkyle et alcoxy sont linéaires ou ramifiés, saturés ou insaturés ; le radical acyle désigne un radical alcanoyle dérivé d'un acide carboxylique aliphatique ayant de 5 à 10 atomes de carbone ou un groupe aroyle dérivé d'un acide carboxylique aromatique.

4. Procédé selon l'une quelconque des revendications 1 à 3, pour la préparation de mono- ou de dihydroxyindoles de formule (II) suivante : 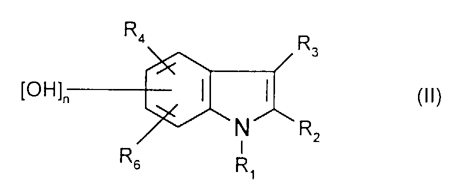 dans laquelle :
- n est un nombre entier égal à 1 ou 2 ;
- R₁ représente un atome d'hydrogène ou un radical alkyle en C₁-C_{4 ;}
- R₂, R₃, R₄ et R₆, identiques ou différents, représentent un atome d'hydrogène, un radical alkyle en C₁-C₈, alcoxy(C₁-C₄)alkyle en C₁-C₄, alcoxy en C₁-C₈, alcoxy(C₁-C₄)alcoxy en C₁-C₄, aryle en C₅-C₁₀, aryloxy en C₅-C₁₀, aryle en C₅-C₁₀ comportant de 1 à 3 hétéroatomes tel qu'un atome d'oxygène ou d'azote, aryloxy en C₅-C₁₀ comportant de 1 à 3 hétéroatomes tel qu'un atome d'oxygène ou d'azote, acyle en C₁-C₈, ou bien un radical pris dans le groupe constitué par les radicaux suivants : 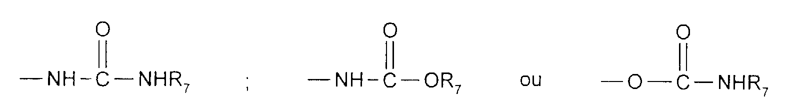 dans lesquels le radical R₇ représente un radical alkyle en C₁-C₈.

5. Procédé selon l'une quelconque des revendications précédentes, pour la préparation du 5,6-dihydroxyindole, du 6-hydroxyindole, du 4-hydroxyindole, du 5-hydroxyindole, du 7-hydroxyindole ou du 5,7-dihydroxyindole.

6. Procédé selon l'une quelconque des revendications précédentes, caractérisé par le fait que l'hydrolase est choisie parmi les lipases, les estérases et les protéases.

7. Procédé selon la revendication 6, caractérisé par le fait que l'hydrolase est fixée sur un support inerte tel que des billes de verre, de la célite, de l'argile ou de la résine.

8. Procédé selon la revendication 6 ou 7, caractérisé par le fait que l'hydrolase est choisie parmi la lipase de Pseudomonas Fluorescens et la lipase de Candida Antartica fixée sur résine.

9. Procédé selon l'une quelconque des revendications précédentes, caractérisé par le fait que la ou les hydrolases représentent de 5 % à 500 % en poids par rapport au poids du composé indolique de départ à faire réagir.

10. Procédé selon la revendication 9, caractérisé par le fait que la ou les hydrolases représentent de 10 % à 100 % en poids par rapport au poids du composé indolique de départ à faire réagir.

11. Procédé selon l'une quelconque des revendications précédentes, caractérisé par le fait que le pH du milieu solvant est compris entre 5 et 9.

12. Procédé selon la revendication 11, caractérisé par le fait que le pH du milieu solvant est compris entre 6,5 et 7,5.

13. Procédé selon la revendication 11 ou 12, caractérisé par le fait que le milieu réactionnel renferme une solution tampon.

14. Procédé selon l'une quelconque des revendications 1 à 12, caractérisé par le fait que le solvant utilisé est un solvant inerte choisi parmi les alcools en C₁-C₄, l'acétonitrile, le tetrahydrofurane, le toluène, l'hexane, l'heptane, le chloroforme et le dichlorométhane.

15. Procédé selon la revendication 14, caractérisé par le fait que le solvant est un alcool en C₁-C₄.

16. Procédé selon l'une quelconque des revendications précédentes, caractérisé par le fait que la température de la réaction est comprise entre 20 et 80°C.

17. Procédé selon la revendication 16, caractérisé par le fait que la température de la réaction est comprise entre 25 et 50°C.

## Patentansprüche

1. Verfahren zur einstufigen Herstellung von Indolverbindungen,
gekennzeichnet durch
Inkontaktbringen mindestens einer Hydrolase, die unter Enzymsubstanzen ausgewählt ist, die unter der Einwirkung von Wasser die Spaltung einer Esterbindung bewirken, mit mindestens einer Indolverbindung, die eine oder zwei über eine Estergruppierung mit dem benzolring verbundene Gruppen aufweist,
in einem Lösungsmittelmedium unter Erhalt eines Monohydroxyindols oder eines Dihydroxyindols.

2. Verfahren nach Anspruch 1, dadurch gekennzeichnet, daß die als Ausgangsverbindungen eingesetzten Indolverbindungen, die eine oder zwei über eine Estergruppierung mit dem Benzolring verbundene Gruppen aufweisen, unter Verbindungen der nachstehenden Formel (I) 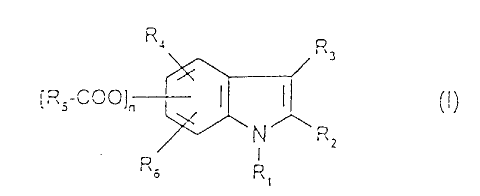 ausgewählt sind,
in der bedeuten:
- n eine ganze Zahl gleich 1 oder 2;
- R₁ ein Wasserstoffatom oder C₁₋₄-Alkyl;
- R₂, R₃, R₄ und R₆, die gleich oder verschieden sind, ein Wasserstoffatom, C₁₋₈-Alkyl, C₁₋₄ -Alkoxy-C₁₋₄-alkyl, C₁₋₈-Alkoxy, C₁₋₄-Alkoxy-C₁₋₄-alkoxy, C₅₋₁₀-Aryl, C₅₋₁₀-Aryloxy, C₅₋₁₀-Aryl mit 1 bis 3 Heteroatomen, wie Sauerstoff oder Stickstoff, C₅₋₁₀-Aryloxy mit 1 bis 3 Heteroatomen, wie Sauerstoff oder Stickstoff, C₁₋₈-Acyl oder eine unter folgenden Gruppen ausgewählte Gruppe: 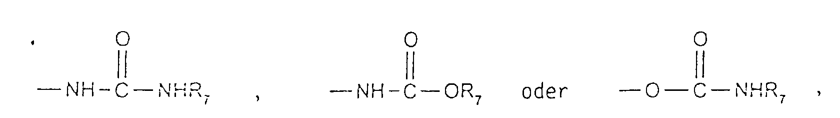 worin R₇ C₁₋₈-Alkyl bedeutet,
und
- R₅ C₁₋₈-Alkyl, C₅₋₁₀-Aryl, C₅₋₁₀-Aryl-C₁₋₈-alkyl, C₁₋₈-Alkyl-C₅₋₁₀-aryl oder C₂₋₈-Acyl-C₅₋₁₀-aryl.

3. Verfahren nach Anspruch 2, dadurch gekennzeichnet, daß in Formel (I) die Alkyl- und Alkoxygruppen geradkettig oder verzweigt und gesättigt oder ungesättigt sind und Acyl eine von einer aliphatischen Carbonsäure mit 5 bis 10 Kohlenstoffatomen abgeleitete Alkanoylgruppe oder eine von einer aromatischen Carbonsäure abgeleitete Aroylgruppe bezeichnet.

4. Verfahren nach einem der Ansprüche 1 bis 3 zur Herstellung von Mono- oder Dihydroxyindolen der nachstehenden Formel (II) : 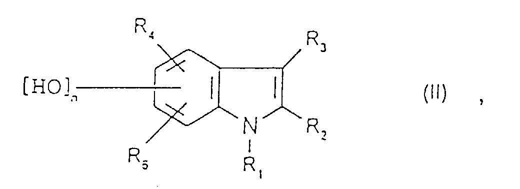 in der bedeuten:
- n eine ganze Zahl gleich 1 oder 2;
- R₁ ein Wasserstoffatom oder C₁₋₄-Alkyl
und
- R₂, R₃, R₄ und R₆, die gleich oder verschieden sind, ein Wasserstoffatom, C₁₋₈-Alkyl, C₁₋₄-Alkoxy-C₁₋₄-alkyl, C₁₋₈-Alkoxy, C₁₋₄ -Alkoxy-C₁₋₄-alkoxy, C₅₋₁₀-Aryl, C₅₋₁₀-Aryloxy, C₅₋₁₀-Aryl mit 1 bis 3 Heteroatomen, wie Sauerstoff oder Stickstoff, C₅₋₁₀-Aryloxy mit 1 bis 3 Heteroatomen, wie Sauerstoff oder Stickstoff, C₁₋₈-Acyl oder eine unter folgenden Gruppen ausgewählte Gruppe: 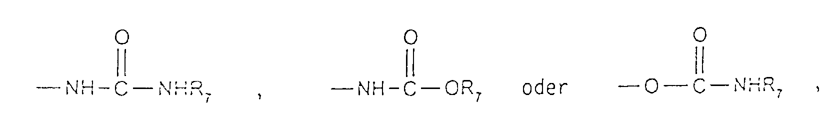 worin R₇ C₁₋₈-Alkyl bedeutet.

5. Verfahren nach einem der vorhergehenden Ansprüche zur Herstellung von 5,6-Dihydroxyindol, 6-hydroxyindol, 4-Hydroxyindol, 5-hydroxyindol, 7-hydroxyindol oder 5,7-Dihydroxyindol.

6. Verfahren nach einem der vorhergehenden Ansprüche, dadurch gekennzeichnet, daß die Hydrolase unter Lipasen, Esterasen und Proteasen ausgewählt wird.

7. Verfahren nach Anspruch 6, dadurch gekennzeichnet, daß die Hydrolase auf einem inerten Träger, wie Glaskugeln, Celit, Tonen oder einem Harz, gebunden ist.

8. Verfahren nach Anspruch 6 oder 7, dadurch gekennzeichnet, daß die Hydrolase unter Lipase von Pseudomonas fluorescens und der an ein Harz gebundenen Lipase von Candida antartica ausgewählt ist.

9. Verfahren nach einem der vorhergehenden Ansprüche, dadurch gekennzeichnet, daß die Hydrolase(n) in einem Mengenanteil von 5 bis 500 Gew.-%, bezogen auf das Gewicht der als Ausgangsverbindung für die Umsetzung eingesetzten Indolverbindung, vorliegen.

10. Verfahren nach Anspruch 9, dadurch gekennzeichnec, daß die Hydrolase(n) in einem Mengenanteil von 10 bis 100 Gew.-%, bezogen auf das Gewicht der als Ausgangsverbindung für die Umsetzung eingesetzten Indolverbindung, vorliegen.

11. Verfahren nach einem der vorhergehenden Ansprüche, dadurch gekennzeichnet, daß der pH-Wert des Lösungsmittelmediums im Bereich von 5 bis 9 liegt.

12. Verfahren nach Anspruch 11, dadurch gekennzeichnet, daß der pH-Wert des Lösungsmittelmediums im Bereich von 6,5 bis 7,5 liegt.

13. Verfahren nach Anspruch 11 oder 12, dadurch gekennzeichnet, daß das Reaktionsmedium eine Pufferlösung umfaßt.

14. Verfahren nach einem der Ansprüche 1 bis 12, dadurch gekennzeichnet, daß das eingesetzte Lösungsmittel ein inertes Lösungsmittel ist, das unter C₁₋₄-Alkoholen, Acetonitril, Tetrahydrofuran, Toluol, Hexan, Heptan, Chloroform und Dichlormethan ausgewählt ist.

15. Verfahren nach Anspruch 14, dadurch gekennzeichnet, daß das Lösungsmittel ein C₁₋₄-Alkohol ist.

16. Verfahren nach einem der vorhergehenden Ansprüche, dadurch gekennzeichnet, daß die Temperatur bei der Umsetzung im Bereich von 20 bis 80 °C liegt.

17. Verfahren nach Anspruch 16, dadurch gekennzeichnet, daß die Temperatur bei der Umsetzung im Bereich von 25 bis 50 °C liegt.

## Claims

1. Process for the preparation of indole compounds in a single step, characterized in that it consists in placing in contact, in a solvent medium, at least one hydrolase chosen from enzymatic substances which, under the action of water, lead to the cleavage of an ester bond, with at least one indole compound bearing one or two radicals connected by an ester function to the benzene ring, thereby obtaining a mono- or a dihydroxy-indole.

2. Process according to Claim 1, characterized in that the starting indole compounds bearing one or two radicals connected by an ester function to the benzene ring are chosen from the compounds of formula (I) below: 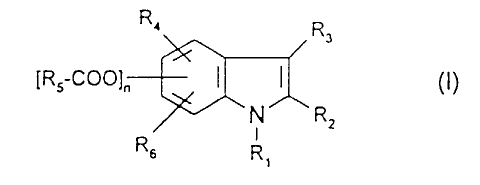 in which:
- n is an integer equal to 1 or 2;
- R₁ represents a hydrogen atom or a C₁-C₄ alkyl radical;
- R₂, R₃, R₄ and R₆, which may be identical or different, represent a hydrogen atom, a C₁-C₈ alkyl radical, a (C₁-C₄)alkoxy(C₁-C₄)alkyl radical, a C₁-C₈ alkoxy radical, a (C₁-C₄)alkoxy(C₁-C₄)alkoxy radical, a C₅-C₁₀ aryl radical, a C₅-C₁₀ aryloxy radical, a C₅-C₁₀ aryl radical containing from 1 to 3 hetero atoms, such as an oxygen or nitrogen atom, a C₅-C₁₀ aryloxy radical containing from 1 to 3 hetero atoms, such as an oxygen or nitrogen atom, a C₁-C₈ acyl radical or alternatively a radical taken from the group consisting of the following radicals: 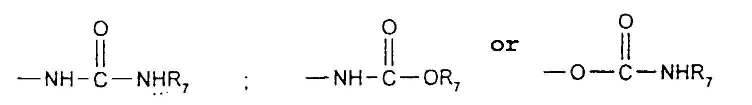 in which the radical R₇ represents a C₁-C₈ alkyl radical;
- R₅ represents a C₁-C₈ alkyl radical, a C₅-C₁₀ aryl radical, a (C₅-C₁₀)aryl(C₁-C₈) alkyl radical, a (C₁-C₈) alkyl(C₅-C₁₀)aryl radical or a (C₂-C₈)acyl-(C₅-C₁₀)aryl radical.

3. Process according to Claim 2, characterized in that, in formula (I), the alkyl and alkoxy radicals are linear or branched, and saturated or unsaturated; the acyl radical denotes an alkanoyl radical derived from an aliphatic carboxylic acid having from 5 to 10 carbon atoms or an aroyl group derived from an aromatic carboxylic acid.

4. Process according to any one of Claims 1 to 3, for the preparation of mono- or dihydroxyindoles of formula (II) below: 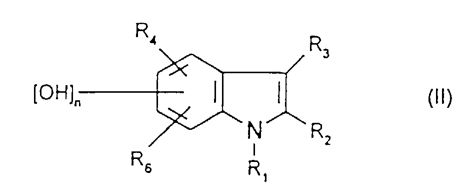 in which:
- n is an integer equal to 1 or 2;
- R₁ represents a hydrogen atom or a C₁-C₄ alkyl radical;
- R₂, R₃, R₄ and R₆, which may be identical or different, represent a hydrogen atom, a C₁-C₈ alkyl radical, a (C₁-C₄)alkoxy(C₁-C₄)alkyl radical, a C₁-C₈ alkoxy radical, a (C₁-C₄)alkoxy(C₁-C₄)alkoxy radical, a C₅-C₁₀ aryl radical, a C₅-C₁₀ aryloxy radical, a C₅-C₁₀ aryl radical containing from 1 to 3 hetero atoms, such as an oxygen or nitrogen atom, a C₅-C₁₀ aryloxy radical containing from 1 to 3 hetero atoms, such as an oxygen or nitrogen atom, a C₁-C₈ acyl radical or alternatively a radical taken from the group consisting of the following radicals: 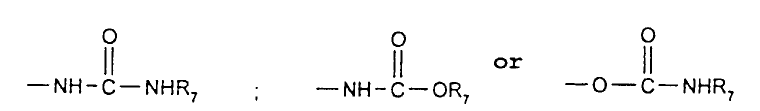 in which the radical R₇ represents a C₁-C₈ alkyl radical.

5. Process according to any one of the preceding claims, for the preparation of 5,6-dihydroxyindole, 6-hydroxyindole, 4-hydroxyindole, 5-hydroxyindole, 7-hydroxyindole or 5,7-dihydroxyindole.

6. Process according to any one of the preceding claims, characterized in that the hydrolase is chosen from lipases, esterases and proteases.

7. Process according to Claim 6, characterized in that the hydrolase is bound to an inert support such as glass beads, Celite, clay or resin.

8. Process according to Claim 6 or 7, characterized in that the hydrolase is chosen from the lipase from Pseudomonas fluorescens and the lipase from Candida antarctica bound to resin.

9. Process according to any one of the preceding claims, characterized in that the hydrolase or hydrolases represent from 5 % to 500 % by weight relative to the weight of the starting indole compound to be reacted.

10. Process according to Claim 9, characterized in that the hydrolase or hydrolases represent from 10 % to 100 % by weight relative to the weight of the starting indole compound to be reacted.

11. Process according to any one of the preceding claims, characterized in that the pH of the solvent medium is between 5 and 9.

12. Process according to Claim 11, characterized in that the pH of the solvent medium is between 6.5 and 7.5.

13. Process according to Claim 11 or 12, characterized in that the reaction medium contains a buffer solution.

14. Process according to any one of Claims 1 to 12, characterized in that the solvent used is an inert solvent chosen from C₁-C₄ alcohols, acetonitrile, tetrahydrofuran, toluene, hexane, heptane, chloroform and dichloromethane.

15. Process according to Claim 14, characterized in that the solvent is a C₁-C₄ alcohol.

16. Process according to any one of the preceding claims, characterized in that the reaction temperature is between 20 and 80°C.

17. Process according to Claim 16, characterized in that the reaction temperature is between 25 and 50°C.
